# EUROPÄISCHE PATENTANMELDUNG

(11) **EP 2 703 375 A1**
(43) Veröffentlichungstag der Anmeldung: **05.03.2014**
(21) Anmeldenummer: 13182210.8
(22) Anmeldetag: 29.08.2013
(51) Int. Cl.: C07C 29/145, C07C 29/56, C07C 45/00

(54) **Verfahren zur Herstellung von Menthol**

(30) Priorität: 31.08.2012 EP 12182667
(71) Anmelder: LANXESS Deutschland GmbH, 50569 Köln (DE)
(72) Erfinder: Mechelhoff, Martin, 50733 Köln (DE); Dreisbach, Claus, 42799 Leichlingen (DE); Jentsch, Jörg-Dietrich, 51381 Leverkusen (DE); Heuer, Lutz, 41542 Dormagen (DE); Wasserscheid, Peter, 91054 Erlangen (DE); Rittsteiger, Anne, 59399 Olfen (DE)

(57) **Zusammenfassung**

Die Erfindung betrifft ein Verfahren zur Herstellung von 2-Isopropyl-5-methylcyclohexanol (D,L-Menthol) über die Hydrierung von Thymol zu Menthon und der anschließenden weiteren Hydrierung zum D,L-Menthol.

## Beschreibung

Die Erfindung betrifft ein Verfahren zur Herstellung von 2-Isopropyl-5-methylcyclohexanol (Menthol) über die Hydrierung von Thymol zu Menthon und der anschließenden weiteren Hydrierung zum Menthol (D,L-Menthol).

2-Isopropyl-5-methylcyclohexanol besitzt drei stereogene Zentren, deshalb gibt es acht Stereoisomere: D,L-Menthol, D,L-Neomenthol, D,L-Isomenthol und D,L-Neoisomenthol.

Unter den natürlich vorkommenden cyclischen Terpenalkoholen nimmt das L-Menthol, der Hauptbestandteil des Pfefferminzöls, aufgrund seiner kühlenden und erfrischenden Wirkung eine Sonderstellung ein. L-Menthol findet daher Verwendung als Riech- oder Geschmackstoff und wird in der Arzneimittelindustrie eingesetzt. Es ist deshalb das wirtschaftlich bedeutendste der Menthol-Stereoisomeren. Daher ist man generell bestrebt, die Hydrierung durch geeignete Wahl der Reaktionsbedingungen und der Katalysatoren so zu führen, dass möglichst viel D,L-Menthol entsteht.

Viele Stoffgemische, deren Komponenten nur geringe Siedepunktsunterschiede aufweisen oder gar Azeotrope bilden, sind durch konventionelle Rektifikation nur schwer bzw. gar nicht trennbar. Dies trifft auf die Trennung von Diastereomeren des 2-Isopropyl-5-methylcyclohexanols aus Stoffgemischen enthaltend zumindest zwei zueinander diastereomere Verbindungen des 2-Isopropyl-5-methylcyclohexanols zu, wie sie typischerweise bei der Hydrierung von Thymol oder nachfolgenden Aufarbeitungsschritten entstehen. Insbesondere die Trennung der Diastereomeren Isomenthol und Menthol ist aufgrund der geringen relativen Flüchtigkeit der beiden Verbindungen zueinander nur unzureichend und unter hohem Energieeinsatz zu leisten.

Die Siedepunkte von D,L-Isomenthol (218,6°C bei 1013 hPa; 75 bis 78°C bei 3,3 hPa) und D,L-Menthol (216,5°C bei 1013 hPa; 75 bis 78°C bei 3,3 hPa) liegen sehr nahe beieinander. Die Trennleistung einer Kolonne bei der destillativen Trennung der einzelnen Menthol-Isomere wird daher insbesondere durch das Verhältnis von D,L-Menthol zu D,L-Isomenthol bestimmt. Für eine hohe Raum-Zeit-Ausbeute an D,L-Menthol bei der destillativen Trennung ist deshalb neben einem möglichst hohen D,L-Menthol-Gehalt im zu trennenden Gemisch auch ein möglichst geringer D,L-Isomenthol-Gehalt nötig. Die Ausbeute an Menthol wird also bei einer gegebenen Destillationskolonne wesentlich durch das Eingangsverhältnis von D,L-Menthol zu D,L-Isomenthol bestimmt.

Zur Herstellung von D,L-Menthol ist es bekannt, Verbindungen, die das Kohlenstoffgerüst des Menthans mit wenigstens einer Doppelbindung besitzen und in 3-Stellung durch Sauerstoff substituiert sind, wie beispielsweise Thymol, in kontinuierlichen Prozessen an festen Katalysatorschüttungen mit Wasserstoff zu hydrieren bzw. Stereoisomere des Menthols an festen Katalysatorschüttungen umzulagern.

In DE 23 14 813 A1 wird ein Verfahren zur Hydrierung von Verbindungen, die das Kohlenstoffgerüst des Menthans mit wenigstens einer Doppelbindung besitzen und in 3-Stellung durch Sauerstoff substituiert sind, an einer Schüttung eines Kobalt-Mangan-Katalysators bei Temperaturen von 170°C bis 220°C und einem Druck von über 25 bar, bevorzugt über 200 bar, beschrieben. In den Beispielen wird bei Temperaturen von 180°C bis 210°C und bei Drucken über 200 bar gearbeitet und dabei ein Gemisch der acht stereoisomeren Menthole erhalten, das zu 59,5 bis 59,9 % aus dem racemischen D,L-Menthol und zu 10,6 bis 10,8 % aus D,L-Isomenthol besteht. Das Menthol-Isomenthol-Verhältnis beträgt dabei maximal 5,7. Durch Modifikation des Kobalt-Mangan-Katalysators mit Kupfer wurden Menthol-Gemische mit D,L-Menthol-Gehalten von 57,6 % und D,L- Isomenthol-Gehalten von 9,2% erreicht, was einem Menthol-Isomenthol-Verhältnis von etwa 6,3 entspricht. Die erhaltenen Gemische weisen jedoch 4 bis 5 % an unerwünschten Nebenprodukte in Form von nicht wiederverwertbaren Kohlenwasserstoffen auf.

Aus EP 0 563 611 A 1 und DE 197 18 116 A 1 ist bekannt, dass die Hydrierung von aromatischen oder teilhydrierten cyclischen Verbindungen, die das Kohlenstoffgerüst des Menthans mit wenigstens einer C=C-Doppelbindung besitzen und in 3-Stellung durch Sauerstoff substituiert sind, mit Wasserstoff an einem Festbettkatalysator durchgeführt werden kann, der Palladium, Ruthenium oder Rhodium oder ein Gemisch dieser Elemente als Aktivbestandteile und Alkalimetallhydroxide und/oder -sulfate als Promotoren jeweils aufgebracht auf einem Träger enthält, wobei der Träger mit einem Metall der Seltenen Erden und Mangan dotiert ist. In den Beispielen wurde bei Temperaturen von 180 bis 240°C und Drucken von 270 bis 300 bar gearbeitet. Dabei wurden Menthol-Gemische erhalten, die ca. 52 bis 57 % D,L-Menthol und 11,5 bis 14,8 % D,L-Isomenthol aufweisen, was einem Menthol-Isomenthol-Verhältnis von 3,6 bis 4,4 entspricht.

In EP 743 296 A 1 werden Katalysatoren offenbart, die aus trägerfreien, verpressten Pulvern von Kobalt-, Mangan- und Erdalkalioxiden bzw. -hydroxiden bestehen und bei Temperaturen von 150°C bis 230°C und Drucken von 25 bis 350 bar eingesetzt werden.

Die Umlagerung von Stereoisomeren des L-Menthols ist in US 5 756 864 beschrieben: Bei Temperaturen von 200 bis 350°C und Wasserstoff-Drucken von 50 bis 350 bar, bevorzugt 100 bis 300 bar wird D-Menthol in einem kontinuierlichen Prozess an einem Katalysator racemisiert und isomerisiert, wobei der Katalysator aus trägerfreien, gepressten Pulvern von Nickel-, Mangan- und Erdalkalihydroxiden bzw. -oxiden besteht. Dabei wurden Menthol-Gemische erhalten, die maximal zu 59,8 % aus D,L- Menthol bestanden.

Aus US 2 843 636 ist bekannt, die Isomerisierung von Stereoisomeren des Menthols zu D,L-Menthol mit Wasserstoff in Gegenwart eines Hydrierkatalysators aus der Gruppe Kupferchromit, Kobalt und Nickel bei 260 bis 280°C und 500 bis 1300 p.s.i.g. (34 bis 90 bar) in Autoklaven durchzuführen. Die entstehenden Gemische wiesen neben ca. 10 bis 12 % D,L-Isomenthol einen D,L-Menthol-Gehalt von 60 bis 64 % auf.

In DE 198 53 562 A wird eine Niederdruck-Hydrierung von Thymol an einer stationären Katalysatorschüttung beschrieben, die einen Temperaturgradienten aufweist: Die ersten zwei von fünf in Serie geschalteten Reaktorrohren werden auf 180°C temperiert, die hinteren drei Reaktorrohre auf 80 bis 90°C. Mit einem Katalysator, der auf einem Träger, der mit einem Metall der Seltenen Erden und mit Mangan dotiert ist, Ruthenium als Aktivbestandteil und Alkalimetallhydroxide als Promotoren enthält, konnte bei einem Druck von 3 bar ein Menthol-Isomerengemisch erhalten werden, das 64,4 Gew-% Menthol und 12,1 % Isomenthol enthielt, was einem Menthol-Isomenthol-Verhältnis von 5,3 entspricht. Die Isomerisierung einer wasserstoffgesättigten Mischung aus D,L-Neomenthol, D,L-Isomenthol und D,L-Menthol lieferte bei Normaldruck ein Isomerengemisch mit einer Zusammensetzung von 65,3 % D,L- Menthol und 12,1 % Isomenthol. Bei diesem Niederdruckverfahren lassen sich hohe Menthol-Gehalte von ca. 65 % erzielen. Das Menthol-Isomenthol-Verhältnis beträgt jedoch maximal 5,4.

In der DE 100 23 283 A wird nun ein verbessertes Verfahren beschrieben, in welchem Isomerengemische, die typischerweise etwa 55% D,L-Menthol aufweisen, durch Isomerisierung mit einfachen Ruthenium-Trägerkatalysatoren Menthol-reichere Gemische herstellen kann, die bis zu 67,3 % D,L-Menthol und lediglich 8,2 % D,L-Isomenthol d.h. ein Menthol-Isomenthol-Verhältnis von bis zu 8,1 aufweisen. Des weiteren ist aus DE 100 23 283 A bekannt, dass die Katalysatoren mit Alkoholaten, Oxiden und Hydroxiden der Alkali- oder Erdalkalimetalle regeneriert werden können.

Allen bekannten Verfahren ist demnach gemeinsam, dass sie nur einen maximalen Anteil von rund 60% an D,L-Menthol ermöglichen, mindestens 8,2 % D,L-Isomenthol ergeben und maximale Menthol-Isomenthol-Verhältnisse von 8,1 erlauben.

Aufgabe der Erfindung war es daher, ein selektives und technisch einfaches Verfahren für die Herstellung von D,L-Menthol in hohen Ausbeuten bereitzustellen, bei dem idealerweise keine oder nur geringe Mengen an D,L-Isomenthol entstehen und welches hohe Menthol-Isomenthol-Verhältnisse erlaubt, wobei gleichzeitig die Bildung unerwünschter Nebenprodukte weitgehend vermieden wird.

Überraschenderweise konnte die Aufgabe durch eine 2-stufige Hydrierung erreicht werden, bei der in der ersten selektiven Hydrierung Thymol zu den Ketonen Iso-/Menthon umgesetzt wird. 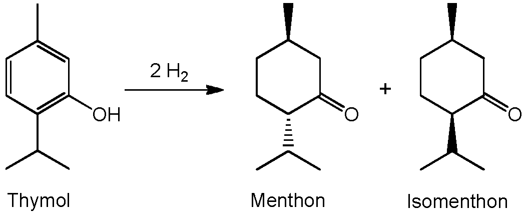 und nach destillativer Trennung der beiden Ketone das erhaltene Menthon dann erneut hydriert wird.

Gegenstand der vorliegenden Erfindung ist ein Verfahren zur Herstellung von 2-Isopropyl-5-methylcyclohexanol (Menthol), dadurch gekennzeichnet, dass
a) Thymol mit Wasserstoff in Gegenwart eines Katalysators, ausgewählt aus der Gruppe der Elemente der Gruppe VIII b (Eisen-Platin-Gruppe) des Periodensystems der Elemente, vorzugsweise Pt, Rh, Ru, Pd, besonders bevorzugt Pd,
   gegebenenfalls in Anwesenheit eines Lösungsmittel hydriert wird,
b) das aus a) isolierte 2-Isopropyl-5-methylcyclohexanon (Menthon) mit Wasserstoff in Gegenwart eines Katalysators, ausgewählt aus der Gruppe VIII b (Eisen-Platin-Gruppe) bevorzugt Pt, Rh, Ru, Pd, besonders bevorzugt Pd, als geträgerte oder nicht geträgerte Katalysatoren, gegebenenfalls in Gegenwart eines Lösungsmittel zu Menthol hydriert wird,
c) das neben Menthol entstehende Neomenthol abgetrennt wird und
e) gegebenenfalls anschließend eine Isomerisierung von Neomenthol zu Menthol durchgeführt wird.

In einer bevorzugten Ausführungsform des erfindungsgemäßen Verfahrens werden die Stufen a) und/oder b) bei Temperaturen von 60°- 200°C, besonders bevorzugt 60 - 120°C und bei einem Druck von mindestens 1,1 bar, bevorzugt >1,1 bis 325 bar, besonders bevorzugt 2 - 100 bar, ganz besonders bevorzugt 10 bis 30 bar durchgeführt.

In einer bevorzugten Ausführungsform der vorliegenden Erfindung wird in Schritt a) auf 1 Mol Thymol ein 2 bis 150 facher molarer Überschuss an Wasserstoff eingesetzt.

In einer bevorzugten Ausführungsform des erfindungsgemäßen Verfahrens werden die Katalysatoren zur Herstellung von Menthon in Schritt a) als geträgerte oder nicht geträgerte, besonders bevorzugt als geträgerte Katalysatoren eingesetzt.

Als Trägermaterialien sind Metalloxide und Aktivkohle bevorzugt. Besonders bevorzugt sind SiO₂, Al₂O₃, TiO₂, ZrO₂ oder Sulfate, und darin vorzugsweise BaSO₄, oder Mischungen daraus und Aktivkohle. Ganz besonders bevorzugt sind Al₂O₃ und Aktivkohle, BaSO₄, Al₂O₃ und/oder Silica. In einer weiteren besonders bevorzugten Ausführungsform der Erfindung ist das Trägermaterial aus Al₂O₃ und Silica und/oder Aktivkohle.

Das Trägermaterial weist vorzugsweise eine BET-Oberfläche von mindestens 100 m²/g, bevorzugt mindestens 160 m²/g, besonders bevorzugt mindestens 180 m²/g auf. Besonders bevorzugt ist Aluminiumoxid, das zusätzlich einem hohen Anteil an makroporösen Poren mit einem Porendurchmesser von mindestens 50 nm aufweist und ein Porenvolumen von mindestens 300 mm³/g, bevorzugt mindestens 600 mm³/g besitzt.

Der Anteil des Katalysators auf dem Trägermaterial beträgt vorzugsweise 0,3 - 10 Gew.-% besonders bevorzugt 2-5 Gew.-%.

Ganz besonders bevorzugt ist ein Trägermaterial aus Al₂O₃ und Silica mit einem Anteil von 2 - 5 Gew.-% Palladium.

Bei den Katalysatoren handelt es sich handelsübliche Katalysatoren, die z.B. bei der Firma Heraeus Materials Technology GmbH & Co. KG oder Johnson Matthey Plc erhältlich sind.

In einer weiteren bevorzugten Ausführungsform der Erfindung wird Schritt a) in einem Lösungsmittel durchgeführt.

Als Lösungsmittel sind cyclische, verzweigte und unverzweigte Alkohole mit 1 - 10 Kohlenstoffatomen, aliphatische und cyclische Ether mit 4 - 12 Kohlenstoffatomen und/oder aliphatische und cycloaliphatische Kohlenwasserstoffe mit 5 - 12 Kohlenstoffatomen, vorzugsweise Methanol, Ethanol, Propanol, Isopropanol, Isobutanol, Tetrahydrofuran, Diethylenglykoldimethylether, Ethylenglykoldimethylether, Tetrahydrofuran, 1,4-Dioxan, Cyclohexan, Methylcyclohexan, Cyclooctan, Hexan, Heptan und/oder Petrolether bevozugt.

Besonders bevorzugt ist Cyclohexan.

Das Verhältnis von Thymol zu Lösungsmittel beträgt vorzugsweise 1: 0 bis 1: 20.

In einer weiteren Ausführungsform der Erfindung kann der in Schritt a) eingesetzte Katalysator rezykliert werden. Hierfür werden bevorzugt kontinuierlich durchströmte Reaktoren eingesetzt, vorzugsweise Wirbelschichtreaktoren oder Reaktoren mit einem festen Katalysatorbett.

Die Abtrennung des in Schritt a) entstandenen Menthons erfolgt vorzugsweise destillativ bei Temperaturen von 50 bis 150°C. Der Destillationssumpf, enthaltend Isomenthon und kleine Anteile an Nebenprodukt, wird vorzugsweise rückgeführt und über eine Keto-Enol-Tautomerie in das thermodynamische Gleichgewicht aus Iso-/Menthon überführt, und erneut destillativ in Menthon und Isomenthon getrennt.

Als Katalysatoren für die Einstellung der Keto-Enol-Tautomerie eignen sich vorzugsweise die Oxide und/oder Hydroxide der Elemente: Aluminium, Magnesium, Eisen, Zink und Silizium. Besonders bevorzugt sind dabei basisches Aluminiumoxid und Magnesiumoxid.

Bei den für die Einstellung der Keto-Enol-Tautomerie eingesetzten Katalysatoren handelt es sich um handelsübliche Katalysatoren, die z.B. bei der Merck KG oder der Lanxess Deutschland GmbH erhältlich sind.

Die Keto-Enol-Tautomerie in das thermodynamische Gleichgewicht aus Iso-/Menthon wird vorzugsweise bei Temperaturen von 0 bis 100° C, besonders bevorzugt bei 20 bis 75°C, durchgeführt.

In einer weiteren bevorzugten Ausführungsform der Erfindung findet diese Umlagerung (die Keto-Enol-Tautomerie) in einem Lösungsmittel statt.

Als Lösungsmittel sind cyclische, verzweigte und unverzweigte Alkohole mit 1 - 10 Kohlenstoffatomen, aliphatische und cyclische Ether mit 4 - 12 Kohlenstoffatomen und/oder aliphatische und cycloaliphatische Kohlenwasserstoffe mit 5 - 12 Kohlenstoffatomen, vorzugsweise Methanol, Ethanol, Propanol, Isopropanol, Isobutanol, Tetrahydrofuran, Diethylenglykoldimethylether, Ethylenglykoldimethylether, Tetrahydrofuran, 1,4-Dioxan, Cyclohexan, Methylcyclohexan, Cyclooctan, Hexan, Heptan und/oder Petrolether bevorzugt.

Besonders bevorzugt ist Cyclohexan.

Das Verhältnis von Thymol zu Lösungsmittel beträgt vorzugsweise 1: 0 bis 1: 20.

In einem weiteren Hydrierschritt (Schritt b)) wird das aus a) isolierte Menthon mit Wasserstoff in Gegenwart eines Katalysators ausgewählt aus der Gruppe VIII b (Eisen-Platin-Gruppe) als geträgerte oder nicht geträgerte Katalysatoren, bevorzugt Pt, Rh, Ru, Pd, besonders bevorzugt Rh gegebenenfalls in Gegenwart eines Lösungsmittel, zu Menthol hydriert, welches im Gemisch mit Neomenthol anfällt:

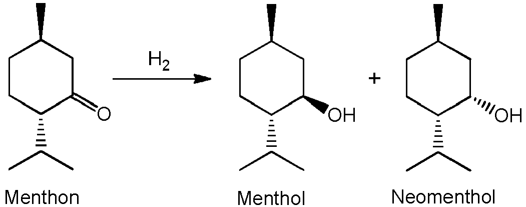

Das nichtumgesetzte Neomenthol kann anschließend rückgeführt und in einer Epimerisierung/Isomerisierung zu Menthol umgesetzt werden.

In einer bevorzugten Ausführungsform der vorliegenden Erfindung wird in Schritt b) auf 1 Mol Menthon ein 2 bis 150 facher molarer Überschuss an Wasserstoff eingesetzt.

In einer bevorzugten Ausführungsform des erfindungsgemäßen Verfahrens werden die Katalysatoren zur Herstellung von Menthon (Schritt b)) als geträgerte oder nicht geträgerte, besonders bevorzugt als geträgerte Katalysatoren eingesetzt.

Als Trägermaterialien sind Metalloxide und Aktivkohle bevorzugt. Besonders bevorzugt sind SiO₂, Al₂O₃, TiO₂, ZrO₂ oder Sulfate, und darin vorzugsweise BaSO₄, oder Mischungen daraus und Aktivkohle. Ganz besonders bevorzugt sind Al₂O₃, Aktivkohle, BaSO₄ und/oder Silica. In einer weiteren besonders bevorzugten Ausführungsform der Erfindung besonders bevorzugt ist das Trägermaterial aus Al₂O₃ und Silica und/oder Aktivkohle.

Das Trägermaterial weist vorzugsweise eine BET-Oberfläche von mindestens 100 m²/g, bevorzugt mindestens 160 m²/g, besonders bevorzugt mindestens 180 m²/g auf. Besonders bevorzugt ist Aluminiumoxid, das zusätzlich einem hohen Anteil an makroporösen Poren mit einem Porendurchmesser von mindestens 50 nm aufweist und ein Porenvolumen von mindestens 300 mm³/g, bevorzugt mindestens 600 mm³/g besitzt.

Der Anteil des Katalysators auf dem Trägermaterial beträgt vorzugsweise 0,3 - 10 Gew.% besonders bevorzugt 2-5 Gew.%.

Ganz besonders bevorzugt ist ein Trägermaterial aus Al₂O₃ mit einem Anteil von 2 - 5 Gew.% Rhuthenium.

Bei den Katalysatoren handelt es sich handelsübliche Katalysatoren, die z.B. bei der Firma Alfa Aesar GmbH erhältlich sind.

Bei der Hydrierung betragen die Temperaturen vorzugsweise von 60° - 200°C, besonders bevorzugt 60 - 120°C.

Bei der Hydrierung beträgt der Druck vorzugsweise mindestens 1,1 bar bis 325 bar, besonders bevorzugt 2 bis 100 bar.

In einer weiteren bevorzugten Ausführungsform der Erfindung wird Schritt c) in einem Lösungsmittel durchgeführt.

Als Lösungsmittel sind cyclische, verzweigte und unverzweigte Alkohole mit 1 - 10 Kohlenstoffatomen, aliphatische und cyclische Ether mit 4 - 12 Kohlenstoffatomen und/oder aliphatische und cycloaliphatische Kohlenwasserstoffe mit 5 - 12 Kohlenstoffatomen, vorzugsweise Methanol, Ethanol, Propanol, Isopropanol, Isobutanol, Tetrahydrofuran, Diethylenglykoldimethylether, Ethylenglykoldimethylether, Tetrahydro-furan, 1,4-Dioxan, Cyclohexan, Methylcyclohexan, Cyclooctan, Hexan, Heptan und/oder Petrolether bevozugt.

Besonders bevorzugt ist Cyclohexan.

Das Verhältnis von Menthon zu Lösungsmittel beträgt vorzugsweise 1: 0 bis 1: 20.

Bei der Hydrierung fällt neben Menthol auch Neomenthol an.

In Schritt c) wird in diesem Gemisch Menthol von Neomenthol abgetrennt. Diese Abtrennung erfolgt vorzugsweise destillativ bei Temperaturen von 60 bis 150°C.

In einer bevorzugten Ausführungsform der vorliegenden Erfindung wird das abgetrennte Neomenthol in einem anschließenden Schritt in einer Isomerisierungsreaktion zu Menthol umgesetzt.

Hierfür werden vorzugsweise die in W02012/010695 beschriebenen Isomerisierungskatalysatoren auf Basis von Ruthenium und Erdalkalimetallalkoxylat eingesetzt, welche auf einem Trägermaterial aus Aluminiumoxid aufgebracht sind.

Als Erdalkalimetallalkoxylate sind Verbindungen der Formel (I),

(R-O)₂M (I)

bevorzugt,
in denen
- R: jeweils unabhängig, vorzugsweise identisch für einen primären, sekundären oder tertiären, cyclischen oder acylischen, verzweigten oder unverzweigten C₁ bis C₂₀-Alkylrest steht, der gegebenenfalls durch Aryl, C₁-C₄-Alkoxyl oder C₆ bis C₁₄-Aryloxy weiter substituiert sein kann und besonders bevorzugt für Methyl, Ethyl, n-Propyl, Isopropyl, n-Butyl, sec.-Butyl, tert. Butyl, n-Pentyl, Neopentyl, n-Hexyl, Cyclohexyl oder die stereoisomeren Methylreste steht und
- M: für Calcium, Strontium oder Barium, vorzugsweise Barium, steht.

Die bevorzugte Bariumalkoxylate können beispielsweise durch Umsetzung von Bariumperchlorat mit den entsprechenden Kaliumalkoxylaten vorzugsweise gelöst im gleichen oder einem anderen Alkohol erhalten werden, wobei sich schwerlösliches Kaliumperchlorat bildet, welches aus den Reaktionslösungen beispielsweise durch Filtration leicht entfernt werden kann.

Bariummentholate sind beispielsweise auch erhältlich durch Versetzen von Bariumethoxid oder Bariumisopropoxid mit einem Überschuß an Menthol-Stereoisomeren und längerem Stehenlassen oder Erwärmen.

Besonders bevorzugt finden Bariumethoxid, 10% w/v in Ethanol, Bariumisopropoxid als feste Substanz, gelöst in Mentholisomeren oder Bariumisopropoxid, 20% w/v in Isopropanol Verwendung.

Das als Trägermaterial eingesetzte Aluminiumoxid kann in allen bekannten Modifikationen, bevorzugt in der γ-Modifikation eingesetzt werden. Vorteilhaft weist das als Trägermaterial eingesetzte Aluminiumoxid eine BET-Oberfläche von mindestens 100 m²/g, bevorzugt mindestens 160 m²/g, besonders bevorzugt mindestens 180 m²/g auf. Besonders bevorzugt ist Aluminiumoxid, das zusätzlich einem hohen Anteil an makroporösen Poren mit einem Porendurchmesser von mindestens 50 nm aufweist und ein Porenvolumen von mindestens 300 mm³/g, bevorzugt mindestens 600 mm³/g besitzt. Als geeignete Trägermaterialien seien beispielhaft die kommerziell erhältlichen Aluminiumoxide SPH 1515, SPH 531, SPH 501 der Firma Rhodia, D 10-10 der Firma BASF und SA 6176 der Firma Norton genannt.

Das Trägermaterial kann beispielsweise in Form von Pulver mit Korngrößen von 0,001 bis 0,1 mm, gebrochenem und gesiebtem Material mit Korngrößen zwischen 0,05 bis 5 mm oder in Formkörpern, vorzugsweise Strangpresslingen, Extrudaten, Pillen, Kugeln oder Granulaten mit Durchmessern von 0,2 bis 30 mm, eingesetzt werden.

Der besondere Vorteil des erfindungsgemäßen Verfahrens ist, dass in effizienter Weise Mischungen von Diastereomeren der 2-Isopropyl-5-methylcyclohexanolen derart getrennt werden können, dass Menthol aus den Diastereomeren Menthol und Neomenthol mit hoher Reinheit erhalten wird.

In dem erfindungsgemäßen Verfahren kann der spezifische Energieverbrauch deutlich gesenkt und die Abmaße der verwendeten Trennapparate, d.h. das notwendige Apparatevolumen je erforderlicher Trennstufe, erheblich reduziert werden.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Beispiele:

### Hydrierung zu Menthon:

### Beispiel 1:

6 g Thymol 40 mmol) wurden mit einem mindestens 2 molaren Überschuss an Wasserstoff in Gegenwart von 3 mol% Kat L1 bis L3 (siehe nachstehende Tabelle) bei Temperaturen von 120°C und bei einem Druck von 10 bar in Gegenwart von Cyclohexan als Lösungsmittel zu 2-Isopropyl-5-methylcyclohexanon (Menthon) hydriert.

| **Katalysator** | **Hersteller** | **Bezeichnung** |
|---|---|---|
| 5 % Pd/Alox | Heraeus | L1 |
| 4 % Pd/Alox-Silica | Heraeus | L2 |
| 2 % Pd/Alox-Silica | Heraeus | L3 |

Mit Heraeus = Heraeus Materials Technology GmbH & Co. KG, Alox= Aluminiumoxid
Silica= Kieselsäure

Das Menthon wurde destillativ bei einer Sumpftemperatur von 133°C abgetrennt.

Die Katalysatoren L1 und L2 zeigten unter den gegebenen Bedingungen eine sehr ähnliche Reaktionsgeschwindigkeit. Vollumsatz wurde nach etwa 30 Min erreicht. Die Aktivität von Katalysator L3 war dagegen deutlich höher. Hier wurde der Vollumsatz bereits nach 5 Minuten erreicht.

Die Katalysatoren L1 und L2 zeigten eine Keton-Selektivität von über 97 % über nahezu den kompletten Umsatzbereich. L3 lieferte bei Vollumsatz sogar noch Werte um 99 %.

Auch bei der Menthon-Selektivität verhielten sich die Katalysatoren L1 und L2 sehr ähnlich. Hier stieg die Menthon-Selektivität über den gesamten Umsatzbereich kontinuierlich bis zum Maximalwert von etwa 68 % an. Bei dem Katalysator L3 stieg die Menthon-Selektivität erst beim Vollumsatz deutlich an.

Bei den Katalysatoren L1 und L2 stieg der Anteil von Isomenthol auf etwa 0,12 %, bei Katalysator L3 konnte kurz vor Erreichen des Vollumsatzes sogar noch ein Wert von 0 % erzielt werden.

### Hydrierung zu Menthon:

### Beispiel 2:

6 g Thymol (40 mmol) wurden mit einem mindestens 2 molaren Überschuss an Wasserstoff in Gegenwart von 2,5 mol% Kat L3 bis L9 (siehe nachstehende Tabelle) bei Temperaturen von 120°C und bei einem Druck von 10 bar in Gegenwart von Cyclohexan als Lösungsmittel zu 2-Isopropyl-5-methylcyclohexanon (Menthon) hydriert.

| **Katalysator** | **Hersteller** | **Bezeichnung** |
|---|---|---|
| 5 % Pd/Alox Typ 325 | Johnsson Matthey Plc | L4 |
| 5 % Pd/Alox | Johnsson Matthey Plc | L5 |
| 5 % Pd/Alox | Johnsson Matthey Plc | L6 |
| 5 % Pd/Alox | Johnsson Matthey Plc | L7 |
| 5 % Pd/Alox | Johnsson Matthey Plc | L8 |
| 5 % Pd/BaSO₄ Typ A201053 | Johnsson Matthey Plc | L9 |
| 2 % Pd/Alox-Silica | Heraeus | L3 |

Mit Heraeus= Heraeus Materials Technology GmbH & Co. KG

Mit allen getesteten Katalysatoren konnten durchgehend sehr gute Selektivitäten zwischen 96 und 98 % erzielt werden. Die besten Ergebnisse konnten mit den Katalysatoren L3, L5 und L9 erreicht werden.

Mit allen getesteten Katalysatoren konnten durchgehend sehr niedrige Isomenthol-Anteile von weniger als 0,17% erzielt werden. Die besten Ergebnisse zeigten die Katalysatoren L3, L6 und L9 mit Isomenthol-Anteile von etwa 0,1 % bei Vollumsatz. Bei Katalysator L3 konnte bis kurz vor Erreichen des Vollumsatzes sogar noch gar kein Isomenthol nachgewiesen werden.

### Beispiel 3:

### Hydrierung von Menthon zu Menthol:

Das in Beispiel 1 Probe L3 entstandene Menthon wurde destillativ bei einer Sumpftemperatur von 133°C abgetrennt und bei 120 °C und 30 bar zu Menthol hydriert. Als Katalysator kam 5 % Ru/Alox reduced von Alfa Aesar zum Einsatz. Die Reaktion wurde im Lösungsmittel Cyclohexan durchgeführt.

Menthon war nach gut 3 h komplett umgesetzt. Neomenthol und Menthol wurden zu etwa gleichen Anteilen gebildet. Der Anteil der unerwünschten Produkte Iso- und Neoisomenthol ist vernachlässigbar klein.

## Patentansprüche

1. Verfahren zur Herstellung von 2-Isopropyl-5-methylcyclohexanol (D,L-Menthol), **dadurch gekennzeichnet, dass**
a) Thymol mit Wasserstoff in Gegenwart eines Katalysators, ausgewählt aus der Gruppe der Elemente der Gruppe VIII b (Eisen-Platin-Gruppe), vorzugsweise Pt, Rh, Ru, Pd, besonders bevorzugt Pd, gegebenenfalls in Anwesenheit eines Lösungsmittels, hydriert wird,
b) das aus a) isolierte 2-Propyl-(2)-5-methylcyclohexanon (Menthon) mit Wasserstoff in Gegenwart eines Katalysators, ausgewählt aus der Gruppe VIII b (Eisen-Platin-Gruppe), bevorzugt Pt, Rh, Ru, Pd, besonders bevorzugt Rh, als geträgerte oder nicht geträgerte Katalysatoren, gegebenenfalls in Gegenwart eines Lösungsmittel zu Menthol hydriert wird, und
c) das neben Menthol entstehende Neomenthol abgetrennt wird und
d) gegebenenfalls anschließend eine Isomerisierungsreaktion von Neomenthol zu Menthol durchgeführt wird, wobei die Stufen a) und/oder c) bei Temperaturen von 60° bis 200°C und bei einem Druck von mindestens 1,1 bar durchgeführt werden.

2. Verfahren nach Anspruch 1, **dadurch gekennzeichnet, dass** als Katalysatoren zur Herstellung von Menthon geträgerte oder nicht geträgerte Katalysatoren eingesetzt werden.

3. Verfahren nach Anspruch 2, **dadurch gekennzeichnet, dass** als Träger für die geträgerten Katalysatoren Metalloxide und Aktivkohle, bevorzugt SiO₂, Al₂O₃, TiO₂, ZrO₂ oder Sulfate, vorzugsweise BaSO₄, oder Mischungen daraus und Aktivkohle, besonders bevorzugt Al₂O₃und Aktivkohle, eingesetzt werden.

4. Verfahren nach einem oder mehreren der Ansprüche 1 bis 3, **dadurch gekennzeichnet, dass** als Lösungsmittel cyclische, verzweigte und unverzweigte Alkohole mit 1 - 10 Kohlenstoffatomen, aliphatische und cyclische Ether mit 4 - 12 Kohlenstoffatomen und/oder aliphatische und cycloaliphatische Kohlenwasserstoffe mit 5 - 12 Kohlenstoffatomen, vorzugsweise Methanol, Ethanol, Propanol, Isopropanol, Isobutanol, Tetrahydrofuran, Diethylenglykoldimethylether, Ethylenglykoldimethylether, Tetrahydrofuran, 1,4-Dioxan, Cyclohexan, Methylcyclohexan, Cyclooctan, Hexan, Heptan und/oder Petrolether, eingesetzt werden.

5. Verfahren nach einem oder mehreren der Ansprüche 1 bis 4, **dadurch gekennzeichnet, dass** die Isomerisierung mit Ruthenium und Erdalkalimetallalkoxylat, welche auf einem Trägermaterial aus Aluminiumoxid aufgebracht wurden, erfolgt.
